# EUROPÄISCHE PATENTANMELDUNG

(11) **EP 1 215 190 A1**
(43) Veröffentlichungstag der Anmeldung: **19.06.2002**
(21) Anmeldenummer: 01126900.8
(22) Anmeldetag: 13.11.2001
(51) Int. Cl.: C07C 45/40, C07C 45/83, C07C 45/80, C07C 45/82, C07C 49/433

(54) **Verbessertes Verfahren zur Reinigung von Ketonen, erhalten aus den korrespondierenden Terpenen durch Ozonolyse und Reduktion**

(30) Priorität: 14.12.2000 AT 20752000
(71) Anmelder: DSM Fine Chemicals Austria Nfg GmbH & Co KG, 4021 Linz (AT)
(72) Erfinder: Giselbrecht, Karlheinz, 4061 Pasching (AT); Schaller, Josef, 4020 Linz (AT); Hermanseder, Rudolf, 4624 Pennewang (AT); Reiter, Klaus, 4020 Linz (AT)
(74) Vertreter: Klostermann, Ingrid

(57) **Zusammenfassung**

Verbessertes, sicheres Verfahren zur Reinigung von Ketonen, erhalten durch Ozonolyse und nachfolgender Reduktion der entsprechenden Terpene, bei welchem im Anschluss an die Ozonolyse und Reduktion von acyclischen, mono-, bi- oder tricyclischen Terpenen mit ozonisierbaren Doppelbindungen, das erhaltene, korrespondierende Roh-Keton mittels Wasserdampfdestillation bei Normaldruck oder bei reduziertem Druck, Extraktion des Wasserdampfdestillates und nachfolgender Destillation in ein hochreines Keton überführt wird.

## Beschreibung

Die Erfindung betrifft ein verbessertes Verfahren, durch welches Terpene mittels Ozonolyse, Reduktion und nachfolgender Aufreinigung in einfacher und sicherer Weise in die entsprechenden korrespondierenden Ketone überführt werden können.

Ketone, die sich von Terpenen ableiten, wie etwa Nopinon, finden beispielsweise in der Riechstoffindustrie oder in der Synthese von chiralen Pharmachemikalien Verwendung.
Die Herstellung dieser Ketone erfolgt gemäß dem Stand der Technik beispielsweise durch Oxidation der entsprechenden Terpene, wie etwa Ocimen, β-Pinen, Limonen, Camphen, Sapinen u.s.w.. Diese Oxidationsmethoden sind jedoch entweder mehrstufig und somit ziemlich aufwendig oder nur mit Hilfe von Schwermetall katalysierten Oxidationsmitteln wie etwa KmnO₄, OsO₄, RuCl₃/NalO₄ u.s.w. durchführbar.
Weiters ist die Herstellung derartiger Ketone mittels Ozonolyse und Reduktion beschrieben. In der Literatur wird jedoch mehrfach vor der destillativen Reinigung des erhaltenen Ketons gewarnt und von Explosionen am Ende der Destillation berichtet. So ist etwa in Chem. Abstr. 114:149283 beschrieben, dass bei der Synthese von Nopinon im großen Maßstab Explosionen auftreten können. So trat nach Ozonisierung von β-Pinen-Proben in CH₂Cl₂/MeOH, anschließendem Abkühlen und Zugabe von AcOH und Zn, um Ozonide zu zerstören, und nachfolgendem Erwärmen auf Raumtemperatur ein heftige Explosion auf. Auch in Chem. Abstr. 114:128191 wird darauf hingewiesen, dass beim Ende der Vakuumdestillation von rohem Nopinon, erhalten durch Ozonolyse von β-Pinen, die Destillationsapparatur explodierte. In J. Org. Chem., Vol. 56, No. 25, 1991 wird ebenfalls auf diese Explosionsgefahr hingewiesen.
Es wird dabei allgemein vermutet, dass der Auslöser dieser Explosionen ein cyclisches Peroxid bzw. Tetroxid oder ein Ozonid ist.

Aufgabe der vorliegenden Erfindung war es, eine Möglichkeit zu finden, durch die Ketone, erhalten durch Ozonolyse und Reduktion der entsprechenden Terpene ,auf einfache und sichere Weise, ohne Explosionsgefahr aufgereinigt werden können.

Unerwarteterweise konnte diese Aufgabe durch die Durchführung einer Wasserdampfdestillation im Anschluss an die Ozonolyse und Reduktion gelöst werden.

Gegenstand der Erfindung ist demnach ein verbessertes, sicheres Verfahren zur Reinigung von Ketonen, erhalten durch Ozonolyse und nachfolgender Reduktion der entsprechenden Terpene, das dadurch gekennzeichnet ist, dass im Anschluss an die Ozonolyse und Reduktion von acyclischen, mono-, bi- oder tricyclischen Terpenen mit ozonisierbaren Doppelbindungen, das erhaltene, korrespondierende Roh-Keton mittels Wasserdampfdestillation bei Normaldruck oder bei reduziertem Druck, Extraktion des Wasserdampfdestillates und nachfolgender Destillation in ein hochreines Keton überführt wird.

Bei dem erfindungsgemäßen Verfahren wird ein Roh-Keton auf einfache und insbesondere sichere Weise aufgereinigt. Das zu reinigende Keton wird dabei durch Ozonolyse mit nachfolgender Reduktion des entsprechenden Terpens erhalten.
Als Ausgangsverbindung eignen sich dabei acyclische, mono-, bi- oder tricyclische Terpene, die eine ozonisierbare Doppelbindung aufweisen. Dies sind beispielsweise aus der Gruppe der acyclischen Terpene Ocimen, Myrcen u.s.w. Bei den mono-, bioder tricyclischen Terpenen kommen bevorzugt solche Terpene in Frage, die eine exocyclische Doppelbindung aufweisen. Dies sind beispielsweise β-Phellandren, (+)- oder (-) - Limonen, β-Pinen, Camphen, Sabinen, Limonen, u.s.w.
Bevorzugt sind mono- oder bicyclische Terpene mit einer exocyclischen Doppelbindung. Besonders bevorzugt sind bicyclische Terpene mit einer exocyclischen Doppelbindung, wobei insbesondere β-Pinen bevorzugt ist.

Die Ozonisierung wird gemäß dem Stand der Technik durchgeführt. Die Temperaturen liegen zwischen -80°C bis knapp unterhalb der Explosionsgrenze des verwendeten Lösungsmittels, d.h. in Abhängigkeit vom verwendeten Lösungsmittel bis 100°C. Vorzugsweise beträgt die Temperatur, wiederum in Abhängigkeit vom eingesetzten Lösungsmittel -40 bis +80°C, wobei die Einhaltung einer Temperatur von -20 bis +50°C wiederum besonders bevorzugt ist.
Die Umsetzung des Terpens erfolgt in einem organischen Lösungsmittel, in dem die Ausgangsverbindung gut löslich ist.
Als Lösungsmittel kommen demnach Alkohole, halogenierte Kohlenwasserstoffe, Säuren, Ester oder Mischungen davon in Frage.
Bevorzugte Lösungsmittel sind CH₂Cl₂, Essigsäure, niedere aliphatische Alkohole mit 1 bis 6 C-Atomen, wie Methanol, Ethanol, i-Propanol, u.s.w., wobei die Verwendung von Methanol und Ethanol besonders bevorzugt ist.
Die Eduktkonzentration liegt je nach Reaktionsbedingungen zwischen 0,1 M und 2 M, wobei eine maximale Eduktkonzentration von 0,5 M bevorzugt ist, um eine bei manchen Ausgangsverbindungen, wie etwa bei β-Pinen, eventuell auftretende Nebelbildung zu verhindern. Es ist jedoch möglich die ausozonisierte, peroxidhaltige Lösung nochmals mit bis zu 0,5M Edukt zu vermischen und ohne Nebelbildung erneut auszuozonisieren.
Ozon wird in äquimolarer Menge oder im Überschuss bezogen auf das Terpen, bzw. auf die ozonisierbare Doppelbindung, eingesetzt , wobei bevorzugt die äquimolare Menge an Ozon zugesetzt wird. Bei Verwendung eines Überschusses kommt es am Ende der Ozonolyse zu einem Ozondurchbruch.
Im Anschluss an die Ozonolyse wird, wiederum gemäß dem Stand der Technik, die peroxidhaltige Lösung katalytisch mit H₂ über einen Edelmetallkatalysator oder durch chemische Reduktion etwa mit Sulfiden aufgearbeitet. Bevorzugt erfolgt eine katalytische Hydrierung. Dabei ist es lediglich maßgeblich, dass die peroxidischen Ozonolyseprodukte in einem unter den Reaktionsbedingungen der Hydrierung inerten, organischen Verdünnungsmittel gelöst vorliegen. Unter organischen Verdünnungsmitteln sind dabei übliche, bei der Hydrierung verwendete Verdünnungsmittel zu verstehen, wie beispielsweise aliphatische oder aromatische, gegebenenfalls halogenierte Kohlenwasserstoffe wie Pentan, Hexan, Cyclohexan, Toluol, Xylole, Methylenchlorid, Dichlorethan, Chlorbenzole, Carbonsäureester wie Essigsäuremethyl-, -ethyl-oder - butylester, Ether wie Diethylether, Diisopropylether, Tetrahydrofuran, Ketone wie Aceton, Methylbutylketon, Alkohole wie Methanol, Ethanol, iso-Propanol.
Bevorzugt werden im erfindungsgemäßen Verfahren peroxidische Ozonolyselösungen in einem niederen, aliphatischen Alkohol mit 1 bis 6 C-Atomen, besonders bevorzugt in Methanol oder Ethanol, eingesetzt. Besonders vorteilhaft ist es, die Hydrierung im gleichen Lösungsmittel durchzuführen, das auch für die Ozonolyse verwendet wird.
Zur praktischen Durchführung wird beispielsweise in einem Hydrierreaktor eine Suspension des Katalysators in dem bei der Ozonisierung verwendeten Alkohol, bevorzugt in Methanol oder Ethanol, ganz bevorzugt in Methanol vorgelegt und die bei der Ozonisierung erhaltene Lösung eingespeist.
Als Katalysatoren eignen sich die für Hydrierungen üblicherweise verwendeten Edelmetallkatalysatoren, die in Form von Pulverkontakten mit Trägermaterialien oder ohne Trägermaterial eingesetzt werden können. Bevorzugt werden Palladium- oder Platinkatalysatoren verwendet, insbesondere Platinkatalysatoren ohne Trägermaterial. Bei Pulverkontakten eignen sich als Trägermaterial beispielsweise Kohle, Aluminium, Silikagel oder Kieselgur. Es können auch Monolithkatalysatoren verwendet werden. Bevorzugt werden Platinkatalysatoren, wie etwa ein Adamskatalysator, eingesetzt.
Die Ausbeuten sind beim erfindungsgemäßen Verfahren an sich unabhängig von der eingesetzten Katalysatormenge, jedoch empfiehlt sich zur Erzielung einer ausreichenden Hydriergeschwindigkeit die genannten Katalysatoren, in Edelmetallmengen von 0.01 bis 5 Gew.-%, vorzugsweise von 0.1 bis 2 Gew.-%, bezogen auf die jeweils pro Stunde eingespeiste Gesamtmenge an Ozonisierungsprodukten, vorzulegen.
Die Hydrierung wird so lange fortgesetzt, bis keine Wasserstoffaufnahme mehr feststellbar ist. Bei der Hydrierung werden zur Reduktion der Ozonisierungsprodukte bis maximal äquivalente Mengen an Wasserstoff verbraucht. Die Menge an Wasserstoff, die bei der Hydrierung verwendet werden kann, reicht von 0,7 Moläquivalenten bis zu einem Moläquivalent.
Die Hydrierung erfolgt beim erfindungsgemäßen Verfahren vorteilhafterweise unter praktisch drucklosen Bedingungen. Unter praktisch drucklosen Bedingungen sollen hier Drücke von 1 bis etwa 3 bar verstanden werden, wie das in der Technik üblich ist, um das Eindringen von Luft in den Hydrierreaktor zu verhindern. Es ist aber auch möglich, die Hydrierung bei einem Druck bis zu 20 bar durchzuführen und dadurch die Hydrierungsgeschwindigkeit zu steigern, wobei jedoch die Gefahr der Nebenproduktbildung steigt.
Die reduktive Spaltung verläuft im allgemeinen exotherm und wird bei Temperaturen von -10 bis +150°C, bevorzugt bei +10 bis 70°C und besonders bevorzugt bei Temperaturen im Bereich von +15 bis 50°C durchgeführt.
Vorteilhafterweise wird während der Hydrierung ein pH-Wert von 2 bis 12, bevorzugt von.8 bis 11 eingehalten. Da sich im Verlaufe der Hydrierung saure Nebenprodukte bilden können, kann gegebenenfalls zur Einhaltung des gewünschten pH-Wertes die dosierte Zugabe einer Base, vorteilhafterweise von verdünnter Natronlauge oder alkoholischer KOH, erfolgen.

Nach erfolgter Ozonolyse und Hydrierung erfolgt die erfindungsgemäße Aufarbeitung der Hydrierlösung.
Dazu wird die erhaltene Hydrierlösung gegebenenfalls zuerst durch Zugabe einer geeigneten anorganischen oder organischen Säure, wie etwa Schwefelsäure, HCI, Ameisensäure, Essigsäure, p-Toluolsulfonsäure u.s.w., auf einen pH-Wert von 4 bis 8, bevorzugt von 5 bis 7 eingestellt und durch Abdestillieren bzw. Abdampfen des Lösungsmittels auf ein Drittel bis zu ein Sechstel eingeengt.
Anschließend erfolgt erfindungsgemäß die Wasserdampfdestillation bei Normaldruck oder bei reduziertem Druck von 10 mbar bis Normaldruck. Bevorzugt wird die Wasserdampfdestillation bei Normaldruck durchgeführt.
Dabei wird in die eingedampfte Hydrierlösung Wasserdampf eingeblasen, wobei ein Faktor von 1:5 bis 1:10 bevorzugt ist.
Die Sumpftemperatur bei der Normaldruck-Wasserdampfdestillation liegt bei etwa 100 bis 102°C, die Kopftemperatur bei etwa 90 bis 97°C.
Das Ende der Wasserdampfdestillation ist erreicht, wenn das Destillat einphasig ist. Nach beendeter Wasserdampfdestillation scheidet sich im Sumpfbehälter eine zweite wasserunlösliche organische Phase ab, die die Substanzen enthält, die für die in der Literatur beschriebenen Explosionen verantwortlich sind. Um das Ausfällen dieser exothermen, nicht wasserdampfflüchtigen Substanzen zu verhindern, wird dem zu destillierendem Gemisch vorzugsweise eine praktisch nicht wasserdampfflüchtige Verbindung, in der sich diese gefährlichen Substanzen gut lösen, zugesetzt. Dies sind bevorzugt stark apolare, nicht wasserdampfflüchtige Hochsieder. Besonders bevorzugt wird Weißöl eingesetzt. Die hochsiedende Verbindung wird in einer Menge von bis zu 30 Gew.%, bevorzugt in einer Menge von 5 bis 10% Gew.%, bezogen auf das gewünschte Endprodukt, eingesetzt.
Das erhaltene zweiphasige Destillat wird sodann mit einem geeigneten Extraktionsmittel ein bis 5mal, bevorzugt bis 3mal, extrahiert. Geeignete Extraktionsmittel sind dabei apolare Lösungsmittel wie Ether, beispielsweise Methyl-ter.-butylether (MtBE),Essigester, Toluol, Hexan u.s.w. Bevorzugt wird MtBE eingesetzt.
Anschließend, gegebenenfalls nach Vereinigung der organischen Phasen, wird das Extraktionsmittel bei Normaldruck oder bei vermindertem Druck bis 20 mbar, bevorzugt bis 10 mbar abdestilliert. Der verbleibende Rückstand, das gewünschte Keton, wird sodann durch eine abschließende Vakuum- oder Normaldruckdestillation aufgereinigt.
Die derart hergestellten, bzw. erfindungsgemäß isolierten und aufgereinigten Ketone werden dabei in Reinheiten von über 99% und in Ausbeuten zwischen 75 und 90% auf sichere und einfache Weise, ohne Explosionsgefahr erhalten.

Besonders bevorzugt wird das erfindungsgemäße Verfahren zur Herstellung von Nopinon aus β-Pinen eingesetzt, wobei sowohl die Ozonolyse als auch die Hydrierung im gleichen C₁-C₆-Alkohol, bevorzugt in Methanol oder Ethanol, durchgeführt wird.

### Beispiel 1:

### a) Ozonolyse:

2 Liter einer 1,0 M methanolische β-Pinen Peroxidlösung wurden durch Ozonolyse einer 1,0 M methanolischen β-Pinen Lösung bei -15°C hergestellt, wobei die Ozonkonzentration im Sauerstoff/Ozon Gasstrom 60 g/m3 betrug. Der Ozonverbrauch entsprach genau einem Moläquivalent (100% d.Th.).

### b) Hydrierung:

Die 2 I 1,0 M Peroxidlösung aus der obigen Ozonolyse wurde bei 20°C und einem pH Wert von 10 mit Wasserstoff bei einem Druck von 50 mbar Ü über Pt (Adams) reduziert. Der Laugenverbrauch für pH Einstellung mit 15% KOH in Methanol betrug 11 ml/l PO-Lösung. Der H2-Verbrauch betrug 85% d.Th.

### c) Aufarbeitung:

2 Liter methanolische Hydrierlösung wurden mit verdünnter Schwefelsäure auf pH 6 gestellt und am am Rotavapor durch Abdampfen von Methanol bei Normaldruck auf 400 g eingeengt. Im Destillat befanden sich noch 4 g/l Nopinon. Der resultierende Rückstand (Suspension) wurde mit 400 g Wasser und 50 g Weißöl (ca. 30% auf eingesetztes Pinen) versetzt und anschließend wurde mit dem Dampferzeuger das Roh Nopinon bei Normaldruck wasserdampfdestilliert (Sumpftemperatur 100°C und Kopftemperatur 97°C). Das Ende der Wasserdampfdestillation war erreicht, als das Destillat einphasig wurde und der Gehalt an Nopinon < 1 g/l Destillat betrug. In Summe erhielt man 2740 g zweiphasiges Destillat. Der Rückstand aus der Wasserdampfdestillation war zweiphasig, wobei in der Weißölphase die gesamten brisanten Nebenprodukte (Exothermie der isolierten brisanten Substanzen beträgt ca. 2000 J/g) gelöst waren (auch nach dem Abkühlen auf Raumtemperatur). Durch den Verdünnungseffekt mit Weißöl betrug die Exothermie in der Weißölphase nur mehr 150 J/g.
Nach zweimaliger Extraktion des zweiphasigen Destillats mit je 1000 ml MtBE (Das Hauptnebenprodukt, Formaldehyd, verbleibt in der wässerigen Phase) wurden die organischen Phasen abgetrennt, vereint und am Rotavapor bei Normaldruck das MtBE quantitativ abdestilliert. Im Rückstand verblieben 225 g Roh Nopinon (Ausbeute 81 % d. Th.) mit einem GC Gehalt von 98%. Im Druck DSC Spektrum des Rückstandes war keine Exothermie mehr ersichtlich. Nach der Vakuumdestillation des Roh Nopinons bei 20 mbar und 130 °C Sumpftemperatur wurden 220 g Nopinon mit einem GC-Gehalt von > 99% erhalten (Ausbeute 79 % d.Th.).

## Patentansprüche

1. Verbessertes, sicheres Verfahren zur Reinigung von Ketonen, erhalten durch Ozonolyse und nachfolgender Reduktion der entsprechenden Terpene, **dadurch gekennzeichnet, dass** im Anschluss an die Ozonolyse und Reduktion von acyclischen, mono-, bi- oder tricyclischen Terpenen mit ozonisierbaren Doppelbindungen, das erhaltene, korrespondierende Roh-Keton mittels Wasserdampfdestillation bei Normaldruck oder bei reduziertem Druck, Extraktion des Wasserdampfdestillates und nachfolgender Destillation in ein hochreines Keton überführt wird.

2. Verfahren nach Anspruch 1, **dadurch gekennzeichnet, dass** als Ausgangsverbindung ein Terpen aus der Gruppe Ocimen, Myrcen, β-Phellandren, (+)- oder (-)-Limonen, β-Pinen, Camphen, Sabinen oder Limonen eingesetzt wird.

3. Verfahren nach Anspruch 1, **dadurch gekennzeichnet, dass** die Ozonolyse in einem Alkohol, halogenierten Kohlenwasserstoff, einer Säure, einem Ester oder inMischungen davon, bei einer Temperatur, in Abhängigkeit vom eingesetzten Lösungsmittel, zwischen -80 und +100°C und einer Eduktkonzentration zwischen 0,1 M und 2M durchgeführt wird.

4. Verfahren nach Anspruch 1, **dadurch gekennzeichnet, dass** die Reduktion durch katalytische Hydrierung in einem aliphatischen oder aromatischen, gegebenenfalls halogeniertem Kohlenwasserstoff, in einem Carbonsäureester, einem Ether, einem Keton oder einem Alkohol in Gegenwart eines Edelmetallkatalysators mit 0,7 bis 1 Moläquivalenten an Wasserstoff und bei einem pH-Wert zwischen 2 und 12 erfolgt.

5. Verfahren nach Anspruch 1, **dadurch gekennzeichnet, dass** nach erfolgter Ozonolyse und Hydrierung, die Hydrierlösung gegebenenfalls durch Zugabe einer organischen oder anorganischen Säure auf einen pH-Wert zwischen 4 und 8 gestellt wird und auf ein Drittel bis zu ein Sechstel eingeengt wird.

6. Verfahren nach Anspruch 1, **dadurch gekennzeichnet, dass** die Wasserdampfdestillation bei Normaldruck oder einem reduzierten Druck von bis zu 10 mbar durchgeführt wird.

7. Verfahren nach Anspruch 6, **dadurch gekennzeichnet, dass** der der Reaktionslösung während der Wasserdampfdestillation eine stark apolare, nicht wasserdampfflüchtige, hochsiedende Substanz in einer Menge von bis zu 30 Gew.%, bezogen auf das Endprodukt, zugesetzt wird.

8. Verfahren nach Anspruch 6, **dadurch gekennzeichnet, dass** das durch die Wasserdampfdestillation erhaltene zweiphasige Destillat mit einem Extraktionsmittel 1 bis 5 mal extrahiert wird.

9. Verfahren nach Anspruch 8, **dadurch gekennzeichnet, dass** das Extraktionsmittel bei Normaldruck oder bei vermindertem Druck bis zu 20 mbar von der organischen Phase abdestilliert wird.

10. Verfahren nach Anspruch 1, **dadurch gekennzeichnet, dass** das Rohketon abschließend durch eine Vakuum- oder Normaldruckdestillation aufgereinigt wird, wodurch das entsprechende Keton in Reinheiten von über 99% und in Ausbeuten von 75 bis 90% erhalten wird.
